# EUROPEAN PATENT APPLICATION

(11) **EP 4 520 305 A1**
(43) Date of publication of application: **12.03.2025**
(21) Application number: 23807543.6
(22) Date of filing: 11.05.2023
(51) Int. Cl.: A61F 13/15

(54) **METHOD FOR MANUFACTURING SCREEN FORMING BODY USED FOR FIBER ACCUMULATING DRUM, AND FIBER ACCUMULATING DRUM USING SCREEN FORMING BODY**

(30) Priority: 20.05.2022 JP 2022083385
(71) Applicant: Zuiko Corporation, Ibaraki-shi Osaka 5670082 (JP)
(72) Inventor: SHIMADA, Takahiro, Ibaraki-shi, Osaka 567-0082 (JP)
(74) Representative: Hasegawa, Kan
(86) International application number: PCT/JP2023/017761
(87) International publication number: WO 2023/223939

(57) **Abstract**

Provided are a method of manufacturing a screen forming body used in a fiber deposition drum, with which it is easy for a screen and a reinforcement member to be strongly bonded together, and which has high reliability; and a fiber deposition drum including the screen forming body. A method of manufacturing a screen forming body (200) used in a fiber deposition drum (100) includes: a process of preparing a flat plate-like screen (210b) in which numerous first holes (212) are formed; a process of preparing a flat plate-like reinforcement member (220b) in which numerous second holes (222) are formed; a process of integrating the flat plate-like screen and the flat plate-like reinforcement member by surface bonding; and a process of forming the screen and the reinforcement member that have been integrated into an arc shape by bending the screen and the reinforcement member that have been integrated.

## Description

### TECHNICAL FIELD

The present invention relates to a screen forming body, and more specifically a screen forming body used in a fiber deposition drum that is used to manufacture absorbers. Furthermore, the present invention relates to a fiber deposition drum using the screen forming body.

### BACKGROUND ART

Conventionally, absorber manufacturing apparatuses using a fiber deposition drum have been known. The fiber deposition drum is a rotating drum whose outer peripheral surface is provided with a plurality of forming molds for forming the absorbers. In the absorber manufacturing apparatus, the absorbers are manufactured by layering an absorbent material that is the raw material of the absorbers in the forming molds of the fiber deposition drum and then releasing the layered absorbent material from the forming molds.

The forming molds used in the fiber deposition drum include air-permeable members (screens) disposed in openings in the outer peripheral surface of the fiber deposition drum. The air-permeable members are subjected to external forces in mutually opposite directions when the absorbent material is layered and when the absorbent material is released, so using the air-permeable members on their own carries the risk that the air-permeable members will deform and sustain a fatigue fracture.

To address this, Patent Document 1 (Japanese Patent No. 5210914) describes using a mesh, in which a plurality of first wires and a plurality of second wires that intersect each other are interconnected at their intersecting points, as a reinforcement member to restrain deformation of the air-permeable member, and particularly bonding the parts at the intersecting points of the first wires and the second wires, which have a high rigidity in the mesh, to the air-permeable member to restrain deformation of the air-permeable member.

### CITATION LIST

### Patent Literature

Patent Document 1: Japanese Patent No. 5210914

### SUMMARY OF INVENTION

### Technical Problem

However, using a mesh of first wires and second wires as the reinforcement member and bonding the parts at the intersecting points of the first wires and the second wires to the air-permeable member such as described in Patent Document 1 (Japanese Patent No. 5210914) carry the risk of not being able to strongly bond the reinforcement member and the air-permeable member together because the places where the reinforcement member and the air-permeable member are bonded together are intermittent.

Furthermore, using a mesh combining a plurality of members (a plurality of first wires and a plurality of second wires) as the reinforcement member such as described in Patent Document 1 (Japanese Patent No. 5210914) may result in a nonuniform distribution of the bonds between the air-permeable member and the reinforcement member and, if the distribution of the bonds between the air-permeable member and the reinforcement member is nonuniform, it carries a risk of not being able to strongly bond the reinforcement member and the air-permeable member together.

Furthermore, the air-permeable member and the reinforcement member are formed in an arc shape after they have been bonded together so that the air-permeable member and the reinforcement member will have a shape corresponding to the shape of the outer peripheral surface of the fiber deposition drum, but if the reinforcement member and the air-permeable member are not strongly bonded together due to reasons such as those described above, this carries the risk that problems will occur, such as the air-permeable member and the reinforcement member becoming unbonded during bending.

### Solution to Problem

A method of manufacturing screen forming body of the present invention is a method of manufacturing a screen forming body used in a fiber deposition drum. The method of screen manufacturing the forming body includes: a process of preparing a flat plate-like screen in which numerous first holes are formed; a process of preparing a flat plate-like reinforcement member in which numerous second holes are formed; a process of integrating the flat plate-like screen and the flat plate-like reinforcement member surface bonding; and a process of forming the screen and the reinforcement member that have been integrated into an arc shape by bending the screen and the reinforcement member that have been integrated.

A fiber deposition drum of the present invention includes a screen forming body. The screen forming body includes a plate-like screen in which numerous first holes are formed and a plate-like reinforcement member in which numerous second holes are formed and which is integrated with the screen by surface bonding.

### Advantageous Effects of Invention

In the method of manufacturing the screen forming body of the present invention, the flat plate-like screen and the flat plate-like reinforcement member are surface bonded together, so compared with a case where the reinforcement member is bonded to the screen at points, the screen and the reinforcement member can be strongly bonded together. For that reason, the occurrence of problems such as the screen and the reinforcement member becoming unbonded in the bending process after the integration of the screen and the reinforcement member can be reduced.

Furthermore, the fiber deposition drum of the present invention uses the screen forming body in which the screen and the reinforcement member are surface bonded together, so the screen can be firmly supported by the reinforcement member, and the life of the screen can be prolonged.

### BRIEF DESCRIPTION OF DRAWINGS

FIG. 1 is a schematic view of an absorber manufacturing apparatus including a fiber deposition drum pertaining to an embodiment of the present invention.
FIG. 2 is a schematic exploded perspective view of the fiber deposition drum pertaining to an embodiment of the present invention.
FIG. 3 is an exploded perspective view of a forming mold of the fiber deposition drum of FIG. 2.
FIG. 4 is a schematic view of a flat plate-like screen, in which first holes have been formed and before being surface bonded to a reinforcement member, of a screen forming body of the forming mold of FIG. 3.
FIG. 5 is a schematic view of a flat plate-like reinforcement member, in which second holes have been formed and before being surface bonded to the screen, of the screen forming body of the forming mold of FIG. 3.
FIG. 6 is a manufacturing flowchart showing the manufacturing process of the screen forming body of the forming mold of FIG. 3.

### DESCRIPTION OF EMBODIMENT

An embodiment of a method of manufacturing a screen forming body used in a fiber deposition drum pertaining to the present invention and a fiber deposition drum using the screen forming body will be described below with reference to the drawings.

It will be noted that the embodiment described below is merely an example of the present invention and is not intended to limit the scope of the present invention. It will be understood by persons skilled in the art that various changes may be made to the following embodiment without departing from the spirit and scope of the present invention set forth in the claims.

### (1) Absorber Manufacturing Apparatus

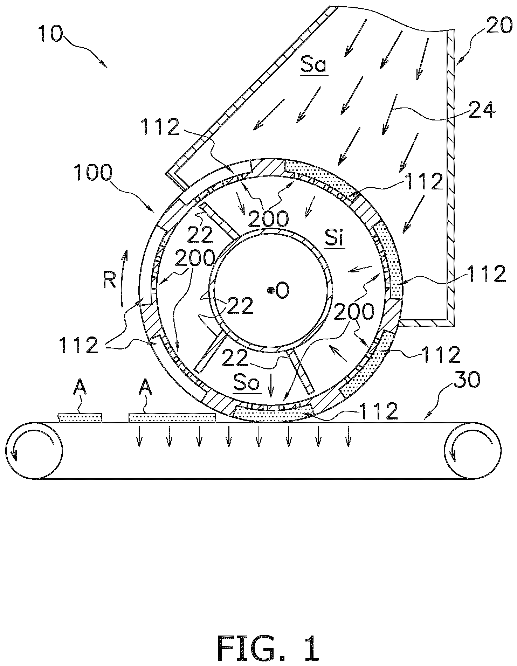

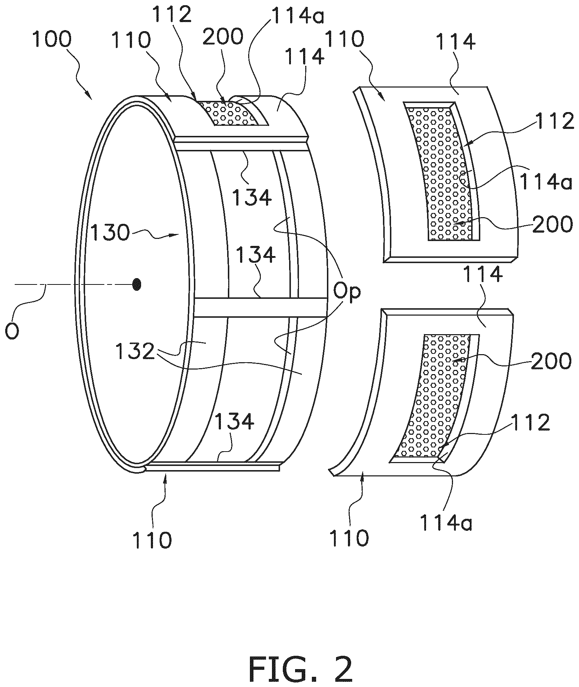

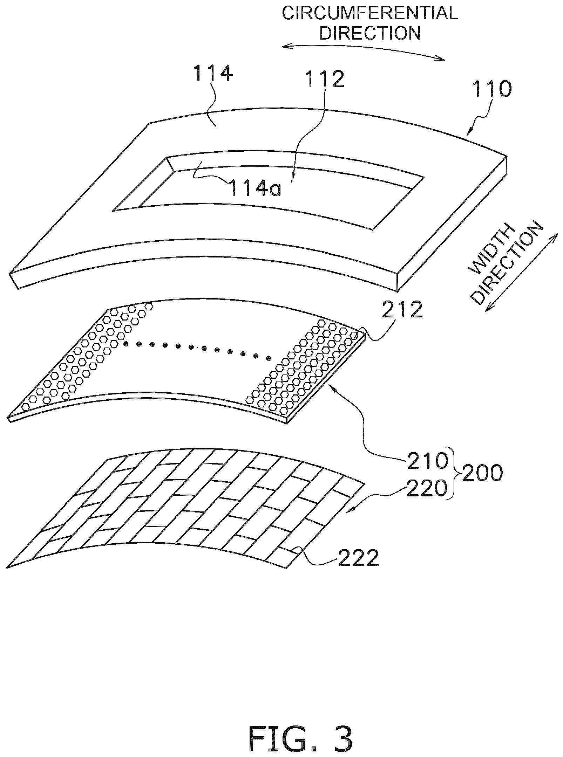

A manufacturing apparatus 10 of an absorber including a fiber deposition drum pertaining to an embodiment of the present invention will now be described with reference to FIG. 1 to FIG. 3. FIG. 1 is a schematic view of the absorber A manufacturing apparatus 10 including a fiber deposition drum 100 pertaining to an embodiment of the present invention. FIG. 2 is a schematic exploded perspective view of the fiber deposition drum 100. FIG. 3 is an exploded perspective view of a forming mold 110 of the fiber deposition drum 100.

The absorber A is an article that is used in disposable diapers and/or sanitary napkins, for example, and absorbs body fluid such as urine and menstrual blood. The absorber A is an article obtained by forming an absorbent material such as pulp fiber into a predetermined shape, though this is not intended to limit the material.

The absorber A manufacturing apparatus 10 mainly includes the fiber deposition drum 100, a duct 20, and a suction conveyor 30 (see FIG. 1).

The fiber deposition drum 100 is a cylindrical rotating drum with a hollow interior. The outer peripheral portion of the fiber deposition drum 100 rotates in the direction of arrow R about an axis of rotation O extending along the center of the cylindrical fiber deposition drum 100 (see FIG. 1).

The fiber deposition drum 100 mainly includes a frame 130, a plurality of forming molds 110, and partition walls 22 (see FIG. 1 and FIG. 2).

The frame 130 includes, as in FIG. 2, a pair of annular frames 132 and a plurality of axial direction frames 134. The annular frames 132 are circular frames centered on the axis of rotation O of the fiber deposition drum 100. The pair of annular frames 132 are disposed apart from each other in the axial direction of the axis of rotation O of the fiber deposition drum 100. Each of the axial direction frames 134 extends along the axial direction of the axis of rotation O of the fiber deposition drum 100 and interconnects the pair of annular frames 132. In the frame 130 are formed openings Op defined by the annular frames 132 and the axial direction frames 134. The plurality of forming molds 110 are attached to the outer peripheral surface of the frame 130 along the circumferential direction thereof so as to close the openings Op (see FIG. 2). The forming molds 110 are attached to the outer peripheral surface of the frame 130 by welding, for example, but the method of attaching the forming molds 110 to the frame 130 is not limited to welding. Each of the forming molds 110 is formed in an arc shape as shown in FIG. 2 when viewed along the axis of rotation O of the fiber deposition drum 100.

Each forming mold 110 mainly includes, as in FIG. 3, a thick plate-like mold body 114 and a screen forming body 200. In each of the forming molds 110, the mold body 114 is disposed on the outer side of the screen forming body 200 in the radial direction of the fiber deposition drum 100. In the central portion of each of the mold bodies 114 is formed a through hole 114a that runs through the mold body 114 in its thickness direction (in the radial direction of the fiber deposition drum 100). Because the through hole 114a is formed in the mold body 114, in each forming mold 110 is formed a recess 112 that is recessed inward in the radial direction of the fiber deposition drum 100 and has the screen forming body 200 as a bottom portion (see FIG. 2). The recess 112 has a shape corresponding to the absorber A that is formed by the manufacturing apparatus 10. For that reason, when the absorbent material that is layered in the recess 112 as described later is removed from the recess 112, the removed absorbent material has the desired shape of the absorber A. It will be noted that although in the present embodiment a single recess 112 is formed in each of the forming molds 110, the number is not limited thereto. For example, a plurality of recesses 112 may be formed in each of the forming molds 110. Furthermore, the shape of the through holes 114a need not be rectangular such as depicted in FIG. 2 and may be another shape.

The screen forming body 200 is a structure where, as in FIG. 3, a plate-like screen 210 formed in an arc shape and a plate-like reinforcement member 220 formed in an arc shape are layered on top of each other in the radial direction of the fiber deposition drum 100 and integrated by surface bonding. The screen 210 is disposed on the outer side of the reinforcement member 220 in the radial direction of the fiber deposition drum 100. In the screen 210, numerous first holes 212 are formed so as to run through the plate that forms the screen 210 (see FIG. 3). In the reinforcement member 220, numerous second holes 222 are formed so as to run through the plate that forms the reinforcement member 220 (see FIG. 3). Because the first holes 212 are formed in the screen 210 and the second holes 222 are formed in the reinforcement member 220, the screen forming body 200 is permeable to air in its thickness direction (in the radial direction of the fiber deposition drum 100). Details about the screen forming body 200 and a method of manufacturing the screen forming body 200 will be described later.

Because the screen forming bodies 200 are disposed in the bottom portions of the recesses 112 of the forming molds 110, the outside of the fiber deposition drum 100 (the space on the outer sides of the forming molds 110) and the inside of the fiber deposition drum 100 (the space on the inner sides of the forming molds 110) are in communication with each other via the screen forming bodies 200 in the bottom portions of the recesses 112.

Inside the fiber deposition drum 100, a plurality of spaces divided by the partition walls 22 disposed inside the fiber deposition drum 100 are formed along the circumferential direction of the fiber deposition drum 100 (see FIG. 1). An intake space Si (see FIG. 1), which is one of the plural spaces, is a space from which inside air is sucked out through openings not shown in the drawings and is a (negative pressure) space whose pressure is lower than the pressure outside the fiber deposition drum 100. The intake space Si takes in air from the space outside the fiber deposition drum 100 via the screen forming bodies 200 disposed adjacent thereto on the radial direction outer side of the intake space Si. An outlet space So (see FIG. 1), which is one of the plural spaces, is a space disposed adjacent to the intake space Si in the circumferential direction of the fiber deposition drum 100 and is disposed on the downstream side of the intake space Si in the rotational direction of the fiber deposition drum 100 (see arrow R in FIG. 1). The outlet space So is a space into which air is sucked through openings not shown in the drawings and is a (positive pressure) space whose pressure is higher than the pressure outside the fiber deposition drum 100. The outlet space So blows out air to the space outside the fiber deposition drum 100 via the screen forming body 200 disposed adjacent thereto on the radial direction outer side of the outlet space So.

It will be noted that when the fiber deposition drum 100 rotates, the frame 130 and the forming molds 110 attached to the frame 130 rotate, but the positions of the spaces inside the fiber deposition drum 100 do not change. For that reason, as the fiber deposition drum 100 rotates, the spaces disposed on the inner sides of each of the forming molds 110 (on the axis of rotation O side of the fiber deposition drum 100) change. More specifically, when the fiber deposition drum 100 rotates, each of the forming molds 110 passes over the outer side of the intake space Si and thereafter pass over the outer side of the outlet space So, and when the fiber deposition drum 100 further rotates, it again passes over the outer side of the intake space Si.

The duct 20 forms, on the radial direction outer side of the fiber deposition drum 100, a supply space Sa that surrounds at least part of the intake space Si inside the fiber deposition drum 100 (see FIG. 1). Air 24 including the absorbent material is supplied to the supply space Sa through an opening not shown in the drawings. When the fiber deposition drum 100 rotates about the axis of rotation O and a certain forming mold 110 moves through the supply space Sa, the air 24 including the absorbent material supplied to the supply space Sa flows into the recess 112 of the forming mold 110 moving through the supply space Sa and flows via the screen forming body 200 into the intake space Si whose pressure is lower than that of the supply space Sa. It will be noted that, as described later, the absorbent material included in the air 24 does not flow into the intake space Si because the first holes 212, which are small in diameter, are formed in the screen 210 of the screen forming body 200. For that reason, the absorbent material included in the air 24 becomes layered in the recess 112 of the forming mold 110 without passing through the screen 210.

The suction conveyor 30 is disposed adjacent to the outlet space So inside the fiber deposition drum 100 via the forming molds 110 (see FIG. 1). The suction conveyor 30 is disposed on the downstream side of the duct 20 (see FIG. 1) in the rotational direction of the fiber deposition drum 100 (see arrow R). The suction conveyor 30 is a conveyor that sucks air through its conveyor surface (conveyance surface) as indicated by the arrows in FIG. 1. The suction conveyor 30 sucks the absorbent material (the absorbers A) that was layered in the recesses 112 of the forming molds 110 when the forming molds 110 moved through the supply space Sa, releases the absorbers A from the recesses 112 of the forming molds 110, and conveys the released absorbers A. The absorbers A conveyed by the suction conveyor 30 are conveyed to a downstream process and are used in disposable diapers and/or sanitary napkins, for example.

### (2) Screen forming body

The screen forming body 200 is, as mentioned above, a structure where the plate-like screen 210 formed in an arc shape and the plate-like reinforcement member 220 formed in an arc shape are layered on top of each other in the radial direction and integrated by surface bonding.

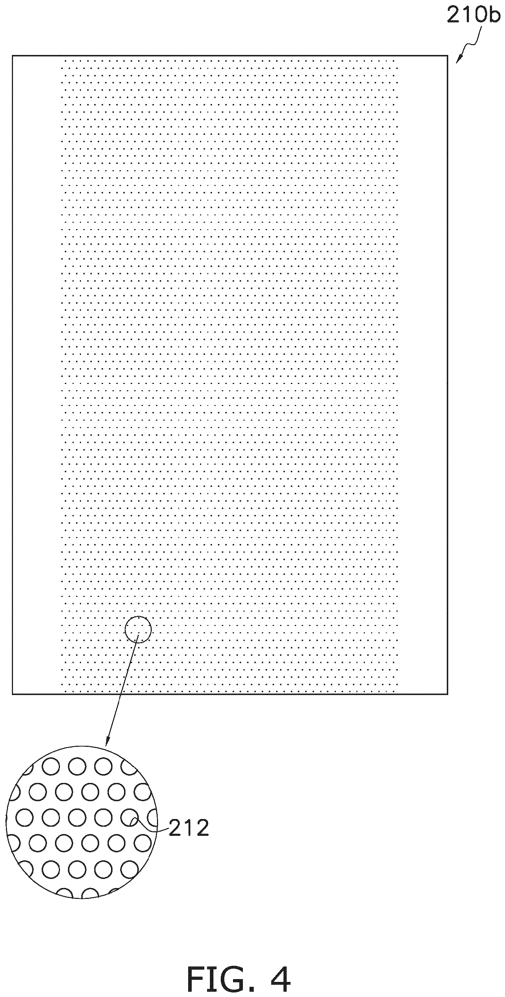

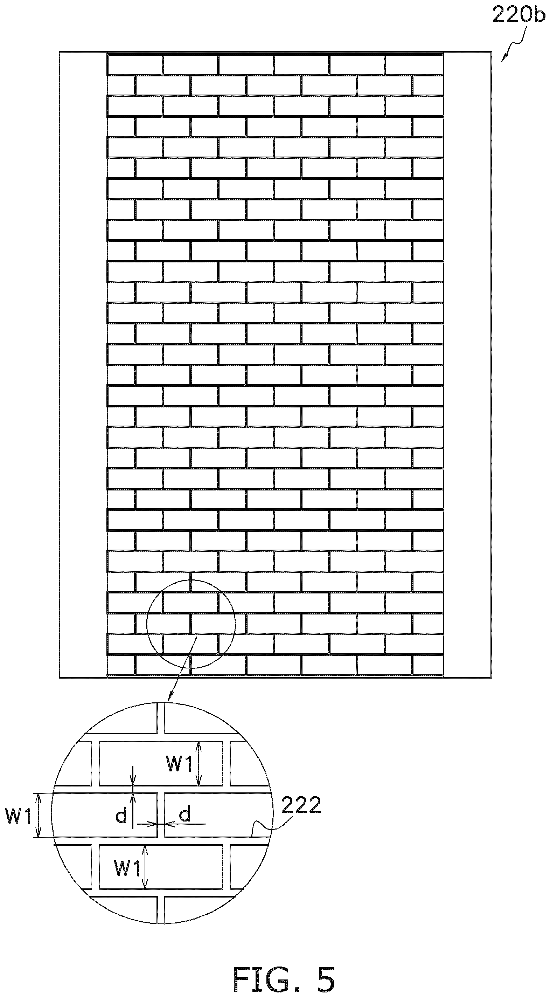

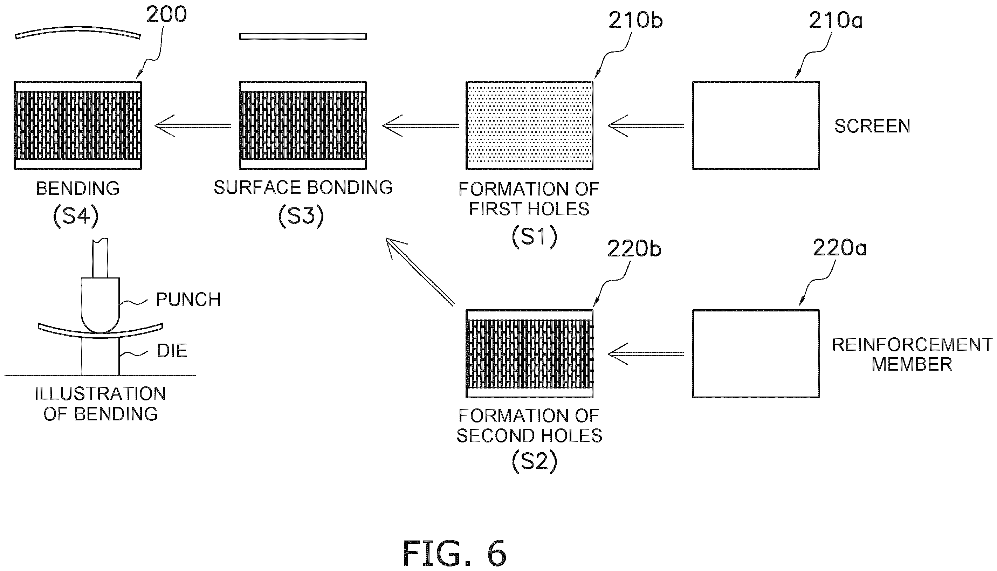

Here, a method of manufacturing the screen forming body 200 and the structures and shapes of the screen and the reinforcement member in the screen forming body 200 during the manufacture of the screen forming body 200 and as a finished product will be described with reference to FIG. 4 to FIG. 6. FIG. 4 is a schematic view of a flat plate-like (prior to bending) screen 210b, in which the first holes 212 have been formed and before being surface bonded to the reinforcement member 220, of the screen forming body 200. FIG. 5 is a schematic view of a flat plate-like (prior to bending) reinforcement member 220b, in which the second holes 222 have been formed and before being surface bonded to the screen 210, of the screen forming body 200. FIG. 6 is a manufacturing flowchart showing the manufacturing process of the screen forming body 200.

The method of manufacturing the screen forming body 200 mainly includes, as shown in FIG. 6, a process of preparing the flat plate-like screen 210b in which the numerous first holes 212 are formed (step S1), a process of preparing the flat plate-like reinforcement member 220b in which the numerous second holes 222 are formed (step S2), a process of integrating the flat plate-like screen 210b and the flat plate-like reinforcement member 220b by surface bonding (step S3), and a process of forming the screen 210b and the reinforcement member 220b that have been integrated into an arc shape by bending the screen 210b and the reinforcement member 220b that have been integrated (step S4). These steps S1 to S4 will be described below.

It will be noted that although step S1 and step S2 are here described in the order of step S1 and then step S2, these steps S1 and S2 may be performed in reverse order or at the same time.

### <Step S1>

The process of preparing the flat plate-like screen 210b in which the numerous first holes 212 are formed (step S1) will now be described.

The flat plate-like screen 210b in which the first holes 212 are formed is manufactured from a flat plate-like member 210a in which the first holes 212 are not formed. The flat plate-like member 210a is a member with a rectangular shape. The flat plate-like member 210a is, for example, a member made of SUS, though this is not intended to limit the material.

Step S1 includes a process of forming the first holes 212 in the flat plate-like member 210a.

The first holes 212 are, as mentioned in the description of the absorber A manufacturing apparatus 10, holes provided to allow air to pass through but without allowing the absorbent material to pass through.

For that reason, the first holes 212 are formed sufficiently small so that the absorbent material does not pass through them. For example, the first holes 212 are circular holes with a diameter of 1 mm or less and more preferably 0.2 to 0.5 mm. It will be noted that the shape of the first holes 212 is not limited to being circular and may be another shape.

Furthermore, in order to guide a sufficient amount of the absorbent material to the recess 112 of the forming mold 110, it is preferred to increase the open area ratio of the flat plate-like member 210a (the ratio of the total area of the first holes 212 formed in the flat plate-like screen 210b to the area of the flat plate-like member 210a). In other words, it is preferred to form numerous first holes 212 in the flat plate-like member 210a. In still other words, it is preferred to form the numerous first holes 212 as close to each other as possible in the flat plate-like member 210a.

In order to form the small-diameter first holes 212 close to each other, the first holes 212 are formed in the flat plate-like member 210a by, for example, etching, though this is not intended to limit the processing method. When the first holes 212 are formed in the flat plate-like member 210a by etching, first, the regions of the flat plate-like member 210a corresponding to the regions where the first holes 212 are not to be formed are masked with a corrosion inhibitor, and then just the regions corresponding to the first holes 212 are corroded with a chemical agent to form the first holes 212.

It will be noted that when the first holes 212 are formed by etching, it is difficult to form the first holes 212 in a flat plate-like member that has a large thickness due to the characteristics of the processing method. For that reason, the thickness of the flat plate-like member 210a is preferably 1 mm or less and more preferably 0.3 mm to 0.6 mm. When the screen 210 is formed by the flat plate-like member 210a that has a thin thickness in this way, the screen 210 is likely to deform and runs the risk of sustaining a fatigue fracture. Thus, here, the screen 210 is used in combination with the reinforcement member 220 as the screen forming body 200.

### <Step S2>

The process of preparing the flat plate-like reinforcement member 220b in which the numerous first holes 212 are formed (step S2) will now be described.

The flat plate-like reinforcement member 220b in which the second holes 222 are formed is manufactured from a flat plate-like member 220a in which the second holes 222 are not formed. The flat plate-like member 220a is a member with a rectangular shape. The flat plate-like member 220a is, for example, a member made of SUS, though this is not intended to limit the material. Because the flat plate-like member 220a is a member whose purpose is to reinforce the screen 210, the thickness of the flat plate-like member 220a is about several mm (e.g., 3 to 5 mm) and thick compared with the flat plate-like member 210a.

Step S2 includes a process of forming the second holes 222 in the flat plate-like member 220a.

The second holes 222 are formed large compared with the first holes 212 because they do not have the purpose of inhibiting the passage of the absorbent material. For example, the second holes 222 are rectangular holes with a length of about 10 to 15 mm and a width of about 5 to 10 mm, though this is not intended to limit the numerical values. It will be noted that, here, what is called the width is the dimension of the second holes 222 in a direction extending in the circumferential direction of the fiber deposition drum 100 when the flat plate-like reinforcement member 220b is finally used in the screen forming body 200, and what is called the length is the dimension of the second holes 222 in a direction extending in the axial direction of the fiber deposition drum 100 when the flat plate-like reinforcement member 220b is finally used in the screen forming body 200. It will be noted that, preferably, the dimensions of widths W1 of the second holes 222 (see FIG. 5) are the same in the numerous second holes 222. By setting the widths W1 of the second holes 222 the same in the flat plate-like reinforcement member 220b, the dimensions of the widths of the numerous second holes 222 in the reinforcement member 220 after bending become substantially the same. By setting the width dimensions of the second holes 222 the same, impedance to the flow of air through the screen forming body 200 tends to be uniform regardless of location, and it becomes easy to manufacture absorbers with a uniform thickness using the fiber deposition drum 100.

It will be noted that it is desired that the total area that the second holes 222 occupy in the flat plate-like reinforcement member 220b (in other words, the open area ratio in the flat plate-like reinforcement member 220b) be as high as possible in a range in which the flat plate-like reinforcement member 220b (in actuality, the reinforcement member 220 after bending) can satisfy its purpose of supporting the screen 210.

Furthermore, the shape of the second holes 222 is not limited to the aforementioned rectangular shape and may be triangular, pentagonal, hexagonal, or another polygonal shape. Furthermore, the shape of the second holes 222 may be circular or elliptical and the like. It will be noted that even when the shape of the second holes 222 is a shape other than rectangular, it is desired that the open area ratio in the flat plate-like reinforcement member 220b be as high as possible in a range in which the flat plate-like reinforcement member 220b can satisfy its purpose of supporting the screen 210.

Furthermore, in order to reduce the adverse effect that the presence of the reinforcement member 220 has on the passage of air in the screen forming body 200, it is preferred that the distance between the second holes 222 that are adjacent to each other be as small as possible in a range that does not impair the purpose of supporting the screen 210. For example, it is preferred that a distance d between the second holes 222 that are adjacent to each other (in other words, the width of the parts that support the screen 210; see FIG. 5) be about 0.3 to 0.7 mm. By setting the distance between the second holes 222 that are adjacent to each other to such a value, the open area ratio of the reinforcement member 220 (the ratio of the total area of the second holes 222 formed in the flat plate-like reinforcement member 220b to the area of the flat plate-like member 220a) can be increased, and the occurrence of a situation where the reinforcement member 220 significantly impedes the flow of air during fiber deposition in the fiber deposition drum 100 can be reduced. At the same time, a minimum distance of 0.3 mm is ensured between the second holes 222 formed in the flat plate-like reinforcement member 220b, so the screen 210 can be firmly supported by the reinforcement member 220.

In order to form such second holes 222, the second holes 222 are formed in the flat plate-like member 220a by laser beam machining, for example, though this is not intended to limit the processing method. By using laser beam machining, the reinforcement member 220 can be precisely formed. As a result, when the screen 210 and the reinforcement member 220 are surface bonded together as described later, the occurrence of problems such as the screen 210 and the reinforcement member 220 being partially not bonded and the bonding strength of those parts declining is easily reduced.

It will be noted that laser beam machining is merely an example of the processing method, and the second holes 222 may be formed by a processing method (e.g., stamping) other than laser beam machining. However, for example, stamping carries the risk of causing burrs in the stamped areas. For that reason, compared with stamping, it is easier to precisely form the reinforcement member 220 using laser beam machining.

### <Step S3>

When the flat plate-like screen 210b is prepared by step S1 and the flat plate-like reinforcement member 220b is prepared by step S2, the flat plate-like screen 210b and the flat plate-like reinforcement member 220b are integrated (step S3).

It will be noted in regard to the integration that although an aspect where just parts of the reinforcement member 220b are bonded to the screen 210b is also conceivable, with such a configuration there is the potential to be unable to strongly bond the flat plate-like screen 210b and the flat plate-like reinforcement member 220b together.

Thus, in the present disclosure, the flat plate-like screen 210b and the flat plate-like reinforcement member 220b are integrated by surface bonding. In other words, the surface of the reinforcement member 220b formed in a mesh shape that faces the screen 210b is substantially entirely bonded to the screen 210b.

The flat plate-like screen 210b and the flat plate-like reinforcement member 220b are surface bonded together by, for example, diffusion bonding, though this is not intended to limit the bonding method. Diffusion bonding is a method in which parent materials are brought into close contact with each other and pressurized to an extent that minimizes plastic deformation as much as possible at a temperature below the melting point of the parent materials and which utilizes atom diffusion occurring in the bonded surfaces to bond the materials together. It will be noted that the method of the surface bonding may also be a method such as cold pressure welding, ultrasonic welding, or forge welding.

### <Step S4>

When the flat plate-like screen 210b and the flat plate-like reinforcement member 220b are integrated in step S3, next, in step S4 processing to form, into an arc shape corresponding to the outer peripheral surface of the fiber deposition drum 100 (the outer peripheral surface of the annular frame 132), the flat plate-like screen 210b and the flat plate-like reinforcement member 220b that are integrated is performed. Specifically, in step S4, bending is performed with respect to the flat plate-like screen 210b and the flat plate-like reinforcement member 220b that are integrated so that the shape of the surface of the reinforcement member 220 after bending on the side not in contact with the screen 210 matches the shape of the outer peripheral surface of the annular frame 132 of the fiber deposition drum 100. In this process, for example, as depicted in FIG. 6, the flat plate-like screen 210b and the flat plate-like reinforcement member 220b are formed into an arc shape by inserting between a punch and a die the flat plate-like screen 210b and the flat plate-like reinforcement member 220b that are integrated.

### (3) Characteristics

### (3-1)

The method of manufacturing the screen forming body 200 of the above embodiment is a method of manufacturing the screen forming body 200 used in the fiber deposition drum 100. The screen forming body 200 manufacturing method includes: the process of preparing the flat plate-like screen 210b in which the numerous first holes 212 are formed; the process of preparing the flat plate-like reinforcement member 220b in which the numerous second holes 222 are formed; the process of integrating the flat plate-like screen 210b and the flat plate-like reinforcement member 220b by surface bonding; and the process of forming the screen 210b and the reinforcement member 220b that have been integrated into an arc shape by bending the screen 210b and the reinforcement member 220b that have been integrated.

In the screen forming body 200 manufacturing method, the flat plate-like screen 210b and the flat plate-like reinforcement member 220b are surface bonded together, so compared with a case where the reinforcement member 220 is bonded to the screen 210 at points, the screen 210 and the reinforcement member 220 can be strongly bonded together. For that reason, the occurrence of problems such as the screen 210 and the reinforcement member 220 becoming unbonded in the bending process after the integration of the flat plate-like screen 210b and the flat plate-like reinforcement member 220b can be reduced.

### (3-2)

In the method of manufacturing the screen forming body 200 of the above embodiment, in the process of integrating the flat plate-like screen 210b and the flat plate-like reinforcement member 220b, the flat plate-like screen 210b and the flat plate-like reinforcement member 220b are diffusion bonded together.

In the screen forming body 200 manufacturing method of the above embodiment, a high bonding strength can be obtained in the bonds of the screen 210 and the reinforcement member 220 by diffusion bonding.

### (3-3)

In the method of manufacturing the screen forming body 200 of the above embodiment, the process of preparing the flat plate-like reinforcement member 220b includes the process of forming the second holes 222 by laser beam machining in the flat plate-like member 220a in which the second holes 222 are not formed.

In the screen forming body 200 manufacturing method of the above embodiment, the reinforcement member 220 can be precisely formed by using laser beam machining. As a result, in the surface bonding of the flat plate-like screen 210b and the flat plate-like reinforcement member 220b, it is easy to reduce the occurrence of problems such as the screen 210b and the reinforcement member 220b being partially not bonded and the bonding strength of those parts declining.

### (3-4)

In the screen forming body 200 manufacturing method of the above embodiment, the distance between the second holes 222 that are adjacent to each other is 0.3 mm to 0.7 mm.

In the screen forming body 200 manufacturing method of the above embodiment, the distance between the second holes 222 formed in the reinforcement member 220 is 0.7 mm or less and narrow, so the open area ratio of the reinforcement member 220 can be increased, and the occurrence of problems such as the reinforcement member 220 significantly impeding the flow of air during fiber deposition in the fiber deposition drum 100 can be reduced. At the same time, in the screen forming body 200 manufacturing method of the above embodiment, a minimum distance of 0.3 mm is ensured between the second holes 222 formed in the reinforcement member 220, so the screen can be firmly supported by the reinforcement member 220.

### (3-5)

In the method of manufacturing the screen forming body 200 of the above embodiment, the width dimensions of the second holes 222 along the circumferential direction when the screen and the reinforcement member have been formed into the arc shape are the same in the numerous second holes 222.

In the screen forming body 200 manufacturing method of the above embodiment, the width dimensions of the second holes 222 in the reinforcement member 220 along the circumferential direction when the screen and the reinforcement member have been formed into an arc shape are the same, so impedance to the flow of air through the screen forming body 200 tends to be uniform regardless of location during fiber deposition in the fiber deposition drum 100. For that reason, absorbers with a uniform thickness are easily manufactured using the fiber deposition drum 100.

### (3-6)

The screen forming body 200 manufacturing method of the above embodiment includes the process of forming the first holes 212 by etching in the flat plate-like member 210a in which the first holes 212 are not formed.

### (3-7)

The fiber deposition drum 100 of the above embodiment includes the screen forming body 200. The screen forming body 200 includes the plate-like screen 210 in which the numerous first holes 212 are formed and the plate-like reinforcement member 220 in which the numerous second holes 222 are formed and which is integrated with the screen 210 by surface bonding.

The fiber deposition drum 100 pertaining to the seventh aspect of the present invention uses the screen forming body 200 in which the screen 210 and the reinforcement member 220 are surface bonded together, so the screen 210 can be firmly supported by the reinforcement member 220, and the life of the screen can be prolonged.

Finally, the technical thought that can be grasped from the above embodiment is appended below.

A method of manufacturing the screen forming body pertaining to a first aspect of the present invention is a method of manufacturing a screen forming body used in a fiber deposition drum. The screen forming body manufacturing method includes: a process of preparing a flat plate-like screen in which numerous first holes are formed; a process of preparing a flat plate-like reinforcement member in which numerous second holes are formed; a process of integrating the flat plate-like screen and the flat plate-like reinforcement member by surface bonding; and a process of forming the screen and the reinforcement member that have been integrated into an arc shape, by bending the screen and the reinforcement member that have been integrated.

In the screen forming body manufacturing method of the first aspect, the flat plate-like screen and the flat plate-like reinforcement member are surface bonded together, so compared with a case where the reinforcement member is bonded to the screen at points, the screen and the reinforcement member can be strongly bonded together. For that reason, the occurrence of problems such as the screen and the reinforcement member becoming unbonded in the bending process after the integration of the flat plate-like screen and the flat plate-like reinforcement member can be reduced.

A method of manufacturing the screen forming body pertaining to a second aspect of the present invention is the method of manufacturing the screen forming body of the first aspect, wherein in the process of integrating the flat plate-like screen and the flat plate-like reinforcement member, the flat plate-like screen and the flat plate-like reinforcement member are diffusion bonded together.

In the screen forming body manufacturing method of the second aspect, a high bonding strength can be obtained in the bonds of the screen and the reinforcement member by diffusion bonding.

A method of manufacturing the screen forming body pertaining to a third aspect of the present invention is the method of manufacturing the screen forming body of the first aspect or the second aspect, wherein the process of preparing the reinforcement member includes a process of forming the second holes by laser beam machining in a flat plate-like member in which the second holes are not formed.

In the screen forming body manufacturing method of the third aspect, the reinforcement member can be precisely formed by using laser beam machining. As a result, in the surface bonding of the flat plate-like screen and the flat plate-like reinforcement member, it is easy to reduce the occurrence of problems such as the screen and the reinforcement member being partially not bonded and the bonding strength of those parts declining.

A method of manufacturing the screen forming body pertaining to a fourth aspect of the present invention is the method of manufacturing the screen forming body of any of the first aspect to the third aspect, wherein a distance between the second holes that are adjacent to each other is 0.3 mm to 0.7 mm.

In the screen forming body manufacturing method of the fourth aspect, the distance between the second holes formed in the reinforcement member is 0.7 mm or less and narrow, so the open area ratio of the reinforcement member can be increased, and the occurrence of problems such as the reinforcement member significantly impeding the flow of air during fiber deposition in the fiber deposition drum can be reduced. At the same time, in the screen forming body manufacturing method of the fourth aspect, a minimum distance of 0.3 mm is ensured between the second holes formed in the reinforcement member, so the screen can be firmly supported by the reinforcement member.

A method of manufacturing the screen forming body pertaining to a fifth aspect of the present invention is the method of manufacturing screen forming body of any of the first aspect to the fourth aspect, wherein width dimensions of the second holes along the circumferential direction when the screen and the reinforcement member have been formed into an arc shape are the same in the numerous second holes.

In the screen forming body manufacturing method of the fifth aspect, the width dimensions of the second holes in the reinforcement member along the circumferential direction when the screen and the reinforcement member have been formed into an arc shape are the same, so impedance to the flow of air through the screen forming body tends to be uniform regardless of location during fiber deposition in the fiber deposition drum. For that reason, absorbers with a uniform thickness are easily manufactured using the fiber deposition drum.

A method of manufacturing the screen forming body pertaining to a sixth aspect of the present invention is the method of manufacturing the screen forming body of any of the first aspect to the fifth aspect, wherein the method includes a process of forming the first holes by etching in a flat plate-like member in which the first holes are not formed.

A fiber deposition drum pertaining to a seventh aspect of the present invention includes a screen forming body. The screen forming body includes a plate-like screen in which numerous first holes are formed and a plate-like reinforcement member in which numerous second holes are formed and which is integrated with the screen by surface bonding.

The fiber deposition drum pertaining to the seventh aspect of the present invention uses the screen forming body in which the screen and the reinforcement member are surface bonded together, so the screen can be firmly supported by the reinforcement member, and the life of the screen can be prolonged.

### REFERENCE SIGNS LIST

- 100: Fiber Deposition Drum

- 200: Screen forming body
- 210: Screen (after bending)
- 210a: Flat Plate-like Member
- 210b: Flat Plate-like Screen
- 212: First Holes
- 220: Reinforcement Member (after bending)
- 220a: Flat Plate-like Member
- 220b: Flat Plate-like Reinforcement Member
- 222: Second Holes

## Claims

1. A method of manufacturing a screen forming body used in a fiber deposition drum, the screen forming body manufacturing method comprising:
a process of preparing a flat plate-like screen in which numerous first holes are formed;
a process of preparing a flat plate-like reinforcement member in which numerous second holes are formed;
a process of integrating the flat plate-like screen and the flat plate-like reinforcement member by surface bonding; and
a process of forming the screen and the reinforcement member that have been integrated into an arc shape by bending the screen and the reinforcement member that have been integrated.

2. The method of manufacturing the screen forming body according to claim 1, wherein in the process of integrating the flat plate-like screen and the flat plate-like reinforcement member, the flat plate-like screen and the flat plate-like reinforcement member are diffusion bonded together.

3. The method of manufacturing the screen forming body according to claim 1 or 2, wherein the process of preparing the reinforcement member includes a process of forming the second holes by laser beam machining in a flat plate-like member in which the second holes are not formed.

4. The method of manufacturing the screen forming body according to any one of claims 1 to 3, wherein a distance between the second holes that are adjacent to each other is 0.3 mm to 0.7 mm.

5. The method of manufacturing the screen forming body according to any one of claims 1 to 4, wherein width dimensions of the second holes along a circumferential direction when the screen and the reinforcement member have been formed into the arc shape are the same in the numerous second holes.

6. The method of manufacturing the screen forming body according to any one of claims 1 to 5, wherein the process of preparing the screen includes a process of forming the first holes by etching in a flat plate-like member in which the first holes are not formed.

7. A fiber deposition drum comprising a screen forming body, wherein the screen forming body includes a plate-like screen in which numerous first holes are formed and a plate-like reinforcement member in which numerous second holes are formed and which is integrated with the screen by surface bonding.
